# EUROPÄISCHE PATENTSCHRIFT

(11) **EP 2 976 363 B1**
(45) Veröffentlichungstag und Bekanntmachung des Hinweises auf die Patenterteilung: **29.04.2020**
(21) Anmeldenummer: 14712255.0
(22) Anmeldetag: 19.03.2014
(51) Int. Cl.: C08B 5/14, C08B 13/00, B01J 13/02

(54) **VERFAHREN ZUR HERSTELLUNG VON SULFATIERTEN CELLULOSEETHERN SOWIE HIERAUS HERGESTELLTE MIKROKAPSELN UND DEREN VERWENDUNG**
METHOD FOR PRODUCING SULPHATED CELLULOSE ETHERS, MICRO-CAPSULES PRODUCED THEREFROM AND USE OF SAID CAPSULES
PROCÉDÉ DE PRODUCTION D'ÉTHERS DE CELLULOSE SULFATÉS, MICROCAPSULES FABRIQUÉES À PARTIR DESDITS ÉTHERS DE CELLULOSE SULFATÉS ET LEUR UTILISATION

(30) Priorität: 19.03.2013 DE 102013204817
(43) Veröffentlichungstag der Anmeldung: 27.01.2016
(73) Patentinhaber: FRAUNHOFER-GESELLSCHAFT zur Förderung der angewandten Forschung e.V., 80636 München (DE)
(72) Erfinder: HETTRICH, Kay, 14548 Schwielowsee (DE); ROHOWSKY, Juta, 14482 Potsdam (DE)
(74) Vertreter: Pfenning, Meinig & Partner mbB
(86) Internationale Anmeldenummer: PCT/EP2014/055505
(87) Internationale Veröffentlichungsnummer: WO 2014/147123

(56) Entgegenhaltungen:
- DE-A1-102005 011 367
- US-A- 3 709 877
- US-A- 3 804 092

## Beschreibung

Die Erfindung betrifft ein Verfahren zur Herstellung von sulfatierten Celluloseestern, die für die Mikroverkapselung eingesetzt werden können. Ebenso betrifft die Erfindung entsprechende Mikrokapseln aus sulfatierten Celluloseestern und kationischen Polymeren. Verwendungen finden diese Mikrokapseln als Arzneimittel, insbesondere als Implantate bzw. Injektionen.

Natrium-Cellulosesulfat (NaCS) ist ein seit langem bekanntes, wasserlösliches Polymer des Schwefelsäurehalbesters der Cellulose. Mit Hilfe einer wässrigen Lösung von NaCS können durch Eintropfen in eine wässrige Lösung kationischer Polymere, wie z. B. Poly-(Diallyldimethylammoniumchlorid) (Poly(DADMAC)), entsprechende Polyelektrolytkomplexe gebildet werden. Dadurch können Materialien, wie z.B. Farbstoffe, Aromen aber auch biologischen Objekte, wie Zellen, Enzyme, Bakterien, verkapselt werden. NaCS wird gebildet durch die Veresterung der Hydroxylgruppen der Cellulose mit einem Sulfatierungsmittel, wie z.B. Schwefelsäureanhydrid, Schwefelsäure oder deren Derivate und der nachfolgenden Umsetzung des aziden Halbesters in ein neutrales Natriumsalz.

Zur Herstellung von NaCS sind grundsätzlich Verfahren bekannt, bei denen die Sulfatierung in heterogener Phase ohne Auflösung des Polymers (heterogen) oder in homogener Phase entweder unter Auflösung des Polymers (quasihomogen) oder nach vorheriger Auflösung des Polymers (homogen) durchgeführt wird.

Lukanoff, B. und Dautzenberg,H. (1994, Das Papier, H eft 6,287-298) entwickelten ein bekanntes heterogenes Herstellungsverfahren (US-PS 2 539 451; US-PS 2 969 355) unter der Verwendung von Schwefelsäure und Propanol als Reaktionsmedium und Sulfatierungsmittel weiter. Für ein solches heterogenes Herstellungsverfahren wird z.B. nach Bohlmann et al. (2002, Chemie Ingenieur Technik, 74, 359-363) zunächst das Reaktionsmedium aus 96%iger Schwefelsäure und Isopropanol in einem molaren Verhältnis von 1,8:1 hergestellt. Die Sulfatierung der Cellulose erfolgt hierin bei -5°C über einen Zeitraum von 150 min. Zum Abbruch der Reaktion wird das Reaktionsgemisch von dem gebildeten Celluloseschwefelsäurehalbester mit Alkohol abgetrennt und ausgewaschen. Anschließend wird das gewaschene Produkt mit Natronlauge in das Natriumsalz überführt.

Wesentliche Nachteile dieses heterogenen Sulfatierungsverfahrens von Cellulose bestehen darin, dass es sich um eine schwer kontrollierbare, exotherme Reaktion in heterogener Phase handelt, die zwangsläufig zu Ungleichmäßigkeiten in der Substituentenverteilung entlang und zwischen den Polymerketten führt und so das Löslichkeitsverhalten der erhaltenen Cellulosesulfate beeinträchtigt.

Ein weiterer gravierender Nachteil des heterogenen Herstellungsverfahrens ist der schnelle und starke Kettenlängenabbau der Cellulose während der fortschreitenden Sulfatierung. Zur Reduzierung des Kettenlängenabbaus der Cellulose wird die Sulfatierungsreaktion, z. B. durch Waschschritte, die hinreichend Wärme abführen und so einen weiteren Temperaturanstieg vermeiden, abgebrochen. Dennoch gewinnen Diffusions- und Quellprozesse sowie die morphologische Struktur der Cellulose einen wesentlichen Einfluss auf den Reaktionsablauf, da die Reaktion insgesamt unter Erhaltung einer Festkörperstruktur der Cellulose abläuft.

Um vollständige Wasserlöslichkeit der heterogen hergestellten Cellulosesulfate ohne Abtrennung unlöslicher Anteile im DS-Bereich < 0,8 zu erreichen, wird in DD 295858 A5 und DE 4019116 A1 eine Voraktivierung der Cellulose vorgeschlagen, wobei allerdings trotzdem nur sehr niederviskose Produkte mit maximal 8,5 mPas in 1 %iger wässriger Lösung erhalten werden. Beim Einsatz dieser Cellulosesulfate zur Herstellung von Symplexmikrokapseln ist zu beobachten, dass nur Mikrokapseln mit sehr geringer mechanischer Festigkeit entstehen.

Nach DE 4021049 können höher viskose Cellulosesulfate aus dem anfallenden Reaktionsprodukt isoliert werden, indem durch zusätzliche Verfahrensschritte die wasserunlöslichen Anteile abgetrennt und die enthaltenen löslichen, aber zu niederviskosen Anteile herausgewaschen werden (vgl. Lukanoff, B. und Dautzenberg, H.: 1994, Das Papier, Heft 6, 287-298).

Im Ergebnis führt der heterogene Herstellungsprozess bei Umsetzung der Cellulose bis zur vollständigen Wasserlöslichkeit zu Produkten mit relativ hohem Substitutionsgrad (mindestens DS = 0,7), einer daraus resultierenden inhomogenen Substituentenverteilung und trotz Einsatz von hochmolekularer Ausgangscellulose zu niederviskosen NaCS.

Bei der homogenen Sulfatierung von Cellulose wird gewöhnlich ein in organischen Lösungsmitteln lösliches Cellulosezwischenderivat eingesetzt, wodurch sich der Kettenlängenabbau der Cellulose während der Sulfatierungsreaktion besser unterdrücken lässt. Da die Sulfatierung nach oder unter vollständiger Auflösung der Festkörperstruktur in einem dipolar aprotischen Lösungsmittel abläuft, wird eine gleichmäßigere Substituentenverteilung erreicht. Das Endprodukt hat eine höhere Lösungsviskosität und ist zum Teil schon bei DS-Werten von 0,25 vollständig wasserlöslich.

Beispielsweise wurden bei Einsatz von relativ niedermolekularem Celluloseacetat (DS=2,4; Cuoxam-DP ca. 250) Lösungsviskositäten der synthetisierten NaCS bis nahe 10mPas (Messung einer 2 %-Lösung in 2N NaOH in einem Ubbelohde-Viskosimeter) erhalten (vgl. DE 4435180).

Durch weitere Modifizierung des Herstellungsverfahrens basierend auf der homogenen Veresterung von teilsubstituierten Celluloseacetaten konnte die zielgerichtete Substituierung der OH-Gruppen an den verschiedenen C-Atomen der Anhydroglucoseeinheit der Cellulose erreicht werden. Wagen knecht et al. (DE 4435082; DE 4435180 und Das Papier, 1996, 712-720) beschreiben die regioselektive Sulfatierung an der C2/C3 oder C6 Position, wobei organolösliche Celluloseacetate als Ausgangspolymer eingesetzt wurden.

Wesentliche Nachteile sind die zu niedrigen Polymerisationsgrade der eingesetzten, handelsüblichen Celluloseacetate (Cuoxam-DP ca. 200 bis 350), so dass sich daraus nach dem gegenwärtigen Stand der Technik keine Cellulosesulfate höherer Lösungsviskosität als ca. 10 mPas in 1 %iger wässriger Lösung herstellen lassen. Die Einstellung eines entsprechenden Lösungsviskositäts-Bereichs der erhaltenen NaCS bei gegebenem Ausgangs-DP des Celluoseacetats ist nach wie vor erstrebenswert.

Die Acetosulfatierung von nativer Cellulose als ein Grundprinzip zur Herstellung von Celluloseacetat bzw. Cellulosesulfat durch Mischveresterung ist seit langem bekannt. Dabei wurden als Reagenzien fast ausschließlich Schwefelsäuren mit Essigsäureanhydrid in glacialer Essigsäure als Reaktionsmedium eingesetzt (US-PS 2683143, US-PS 2969356, US-PS 3086007, US-PS 3075963, US-PS 4005251). Anstelle von Schwefelsäure wurde auch Natriumchlorsulfonat eingesetzt (US-PS 2969355). Im Ergebnis der Untersuchungen von Chauvelon (G. Chauvelon et al., Carbohydrate Resarch 338 (2003) 743-750) zur Herstellung wasserlöslicher Celluloseacetatsulfate zeigte sich eine starke Uneinheitlichkeit dieser Heterogenreaktion, so dass das Zielprodukt nur durch Fraktionierung gewonnen werden konnte.

Es ist weiterhin bekannt, dass eine unter Auflösung verlaufende Acetosulfatierung von Cellulose bei Verwendung von N,N-Dimethylformamid als Reaktionsmedium möglich ist. Dabei wurden als Reagenzmischung Acetanhydrid / S03 (Wagenknecht, W. et al., "Cellulosics: materials for selective separations and other technologies", Kennedy, Phillips, Williams, Horwood (1993) 205-211) oder Acetanhydrid / Chlorsulfonsäure (Wagenknecht, W.: Das Papier 50(1996)12,712-720) eingesetzt. Nach alkalischer Abspaltung der instabilen Acetylgruppen wurden bis zu DS-Sulfat von ca. 0,8 ausschließlich in C6-Stellung der Anhydroglucoseeinheit substituierte wasserlösliche Cellulosesulfate erhalten.

Nachteile der bisher auf diesem Wege synthetisierten Cellulosesulfate bestehen in der Ungleichmäßigkeit bei DS<0,6, die zu Heterogenitäten in einer wässrigen Lösung und damit zur Unbrauchbarkeit für die Herstellung von Symplexmembranen bzw. stabilen Polyelektrolytkomplexen führt.

Eine weitere Möglichkeit zur Herstellung von Cellulosesulfat durch Acetosulfatierung wird in EP1863851 beschrieben. Durch entsprechend definierte Neutralisationsbedingungen wird bei der Fällung der Kettenabbau verhindert, der Polymerisationsgrad und damit verbunden die Lösungsviskosität des nach Aufarbeitung erhaltenen Cellulosesulfats fixiert. Der Nachteil der Herstellung von Cellulosesulfat mittels Acetosulfatierung ist in dem größeren präparativen Aufwand, z. B. durch die notwendige Abspaltung der Acetatgruppen, zu sehen.

Die Herstellung von Cellulosesulfat nach Lösung in ionischen Flüssigkeiten, wie 1-Ethyl-3-methyl-imidazoliumacetat (EMIMAc) oder 1-Butyl-3-methyl-imidazoliumchlorid (BMIMCI) ist in DE 10 2007 035 322 beschrieben. Die Erfindung macht infolge der hohen Viskosität den Zusatz von Co-Solvenzen wie N,N-Dimethylformamid (DMF) notwendig. Neben diesen erhöhten präparativen Aufwand ist die Verwendung ionischer Flüssigkeiten als Nachteil zu nennen. Eine Verwendung der Cellulosesulfate für medizinische und pharmazeutische Anwendungen ist aufgrund der Nutzung der ionischen Flüssigkeiten nur nach aufwendigem Reinigungsprozess möglich. Außerdem ist derzeit die Verwendung von ionischen Flüssigkeiten im großtechnischen Maßstab durch deren hohe Herstellungskosten Grenzen gesetzt.

Celluloseether, wie Carboxymethylcellulose (CMC), Hydroxyethylcellulose (HEC), Methylcellulose (MC), Ethylcellulose (EC) oder Hydroxypropylcellulose (HPC) sind in unserem täglichen Leben allgegenwärtig und werden z. B. als potenzieller Filmbildner, Emulgator, Schutzkolloid, Stabilisator, Klebstoff, Waschhilfsmittel oder Verdickungsmittel eingesetzt. So werden Celluloseether z. B. im Haushalt, in kosmetischen Produkten, in der Medizin, in der Nahrungs- und Genussmittelindustrie, in der Textil- und Papierindustrie oder in der Landwirtschaft bereits angewendet. Damit sind diese Produkte großtechnisch verfügbar.

Aufgabe der Erfindung ist es durch möglichst einfache Modifikationen von Polysaccharidethern nach Einführung von anionischen Gruppen Polymere zu erhalten, die zur Verkapselung verschiedenster Materialien geeignet sind. Die Kapseln sollen durch Wechselwirkung mit kationischen Polymeren, wie poly-(DADMAC), erzeugt werden. Aber auch andere Polymere mit entsprechender kationischer Ladung, z. B. Chitosan oder andere kationische synthetische Polymere sind vorstellbar.

Diese Aufgabe wird durch das Verfahren zur Herstellung von sulfatierten Celluloseestern mit den Merkmalen des Anspruchs 1, die sulfatierten Celluloseester mit den Merkmalen des Anspruchs 8, das Verfahren zur Herstellung von Mikrokapseln mit den Merkmalen des Anspruchs 9 und die entsprechend hergestellten Mikrokapseln mit den Merkmalen des Anspruchs 13 gelöst. In Anspruch 14 werden erfindungsgemäße Verwendungen der Mikrokapseln angegeben. Die weiteren abhängigen Ansprüche zeigen vorteilhafte Weiterbildungen auf.

Erfindungsgemäß wird ein Verfahren zur Herstellung von sulfatierten Celluloseestern mit folgenden Schritten bereitgestellt:
a) Lösen mindestens eines Celluloseesters in einem polaren aprotischen Lösungsmittel,
b) Zusatz eines Sulfatierungsmittels zur Lösung des Celluloseesters, wobei die Sulfatierung bei einer Temperatur von -10 bis 150 °C erfolgt,
c) Ausfällung des sulfatierten Zwischenprodukts in einem Fällungsmedium enthaltend Wasser, mindestens einen Alkohol und mindestens ein Natriumsalz,
d) Waschen und Trocknen des Fällungsprodukts unter Erhalt eines Celluloseestersulfats.

Vorzugsweise ist der mindestens eine Celluloseether ausgewählt aus der Gruppe bestehend aus Methylcellulose, Hydroxyethylcellulose, Hydroxypropylcellulose, Ethylcellulose, Methylhydroxyethylcellulose, Methylhydroxypropylcellulose, Methylhydroxybutylcellulose, Ethylhydroxyethylcellulose, Carboxymethylhydroxyethylcellulose, Benzylcellulose oder Mischungen hiervon.

Das polar aprotische Lösungsmittel ist vorzugsweise ausgewählt aus der Gruppe bestehend aus tertiäre Carbonsäureamide, insbesondere Dimethylformamid, Kohlesäureester, insbesondere Dimethylcarbonat, Sulfoxide, insbesondere Dimethylsulfoxid, Lactame, insbesondere N-Methyl-2-pyrrolidon sowie Mischungen hiervon.

Eine bevorzugte Ausführungsform sieht vor, dass das Sulfatierungsmittel ausgewählt ist aus der Gruppe bestehend aus Schwefelsäure, Chlorsulfonsäure, Amidosulfonsäure, SO₃-Komplexe und Mischungen hiervon.

Das Fällungsmedium enthält als Alkohol vorzugsweise Methanol, Ethanol, Propanol, 2-Propanol oder 2-Methyl-2-Propanol und Mischungen hiervon. Das Gemisch enthält dabei vorzugsweise von 0 bis 50 % (w/w) des Alkohols, besonders bevorzugt von 2 bis 20 % (w/w) des Alkohols.

Das Fällungsmedium enthält als Natriumsalz vorzugsweise Natriumacetat, Natriumchlorid, Natriumhydrogencarbonat, Natriumcarbonat, Natriumsulfat oder Mischungen hiervon.

Es ist weiter bevorzugt, dass das Waschen mit einem Alkohol, insbesondere Methanol, Ethanol, Propanol, 2-Propanol oder 2-Methyl-2-Propanol und/oder das Trocknen mittels Gefriertrocknung, Trocknung im Vakuum, Sprühtrocknung und/oder Lösungsmittelaustausch erfolgt.

Erfindungsgemäß wird ebenso ein sulfatierter Celluloseester bereitgestellt, der nach dem zuvor beschriebenen Verfahren herstellbar ist. Dieser sulfatierter Celluloseester weist einen jetzt DS_{(Sulfat)} von 0,1 bis 2,9 und einer Lösungsviskosität im Bereich von 1 bis 1000 mPas bei einer Konzentration des Celluloseestersulfats von 1 % (w/w) in Wasser auf.

Erfindungsgemäß wird ebenso ein Verfahren zur Herstellung von Mikrokapseln aus mindestens einem sulfatierten Celluloseester, wie er zuvor beschrieben wurde, sowie mindestens einem kationischen Polymer bereitgestellt, das folgende Schritte aufweist:
i) Herstellung einer wässrigen Lösung des sulfatierten Celluloseesters, wobei die Konzentration des sulfatierten Celluloseesters im Bereich von 0,5 bis 10 % (w/w) liegt,
ii) Zugabe eines mindestens eines zu verkapselnden Materials,
iii) Vertropfung der Lösung aus ii) in eine kationische Polymerlösung unter Bildung von Mikrokapseln.

Die kationischen Polymerlösung enthält vorzugsweise Dodecylamin, Ethylenamin, Piperazin, Methylblau, Arginin, Triethylamin, Spermin, Polydimethylammonium, Polyvinylbenzyltrimethylammonium, kationische Chitosane, deren Derivate sowie Mischungen hiervon.

Eine weitere bevorzugte Ausführungsform sieht vor, dass mindestens ein zu verkapselndes Material biologischen Ursprungs ist und bevorzugt ausgewählt ist aus der Gruppe bestehend aus nativen oder veränderten Zellen von Mensch und Tier, native oder veränderte Bakterien native oder veränderte Viren, native oder veränderte Hefen, isolierte Proteine oder Proteingemische, Enzymen, Antikörper oder Antikörperbestandteile und/oder Nukleinsäuremoleküle.

Eine weitere bevorzugte Ausführungsform sieht vor, dass mindestens ein zu verkapselndes Material nicht-biologischen Ursprungs ist und bevorzugt ausgewählt ist aus der Gruppe bestehend aus Farbstoffe, Geschmackstoffe, Geschmacksverstärker, Aromen, Duftstoffe, Insektizide, Pestizide, Fungizide, Detergenzien und Kombinationen hiervon.

Erfindungsgemäß werden ebenso Mikrokapseln bereitgestellt, die nach den zuvor beschriebenen Verfahren hergestellt wurden.

Verwendungen finden die erfindungsgemäßen Mikrokapseln als Arzneimittel, insbesondere zur Implantation oder Injektion.

Anhand der nachfolgenden Beispiele soll der erfindungsgemäße Gegenstand näher erläutert werden, ohne sich auf die hier gezeigten spezifischen Ausführungsformen einschränken zu wollen.

### Beispiel 1

5 g (atro) einer Hydroxyethylcellulose (MS = 2,5) wurden in 150 ml N/N-Dimethylformamid (DMF) gegeben und 3 Stunden bei 85°C bis zur vollständigen Lösung gerührt.

Die Sulfatierung wurde durch die Zugabe von 4 ml Schwefelsäure (4 mol/mol AGU) in 40 ml DMF gestartet. Die Synthese erfolgte bei einer Temperatur von 30°C.

Nach 4 Stunden erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1/5 Stunden gerührt. Anschließend wurde filtriert und dreimal mit je 300 ml einer Waschlösung, bestehend aus 4% (w/w) Natriumacetat in Ethanol-Wasser-Gemisch (1 : 1 / w/w), gewaschen. Darauf folgten zwei Wäschen in je 300 ml Ethanol. Abschließend wurde das Produkt in Wasser gelöste, der pHWerte auf 8 eingestellt, über einen Zeitraum von 48 Stunden dialysiert und durch Gefriertrocknung getrocknet.

### Beispiel 2

5 g (atro) einer Hydroxyethylcellulose (MS = 2,5) wurden in 150 ml DMF gegeben und 3 Stunden bei 85°C bis zur vollständigen Lösung gerührt.

Die Sulfatierung wurde durch die Zugabe von 3,6 g Sulfaminsäure (2 mol/mol AGU) gelöst in 30 ml DMF gestartet. Die Synthese erfolgte bei einer Temperatur von 50°C.

Nach 6 Stunden erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1,5 Stunden gerührt. Anschließend wurde filtriert und dreimal mit je 300 ml einer Waschlösung, bestehend aus 4 % (w/w) Natriumacetat in Ethanol-Wasser-Gemisch (1: 1, w/w), gewaschen. Darauf folgten zwei Wäschen in je 300 ml Ethanol. Abschließend wurde das Produkt in Wasser gelöste, der pH-Werte auf 8 eingestellt und über einen Zeitraum von 48 Stunden dialysiert und durch Gefriertrocknung getrocknet.

### Beispiel 3

5 g (atro) einer Hydroxypropylcellulose (MS = 2,8) wurden in 150 ml DMF gegeben und 3 Stunden bei 85°C bis zur vollständigen Lösung gerührt.

Die Sulfatierung wurde durch die Zugabe von 2,45 g Schwefelsäuretrioxid-Pyridin-Komplex (1 mol/mol AGU) gelöst in 50 ml DMF gestartet. Die Synthese erfolgte bei einer Temperatur von 60°C.

Nach 2 Stunden erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1,5 Stunden gerührt. Anschließend wurde filtriert und dreimal mit je 300 ml einer Waschlösung, bestehend aus 4% (w/w) Natriumacetat in Ethanol-Wasser-Gemisch (1: 1, w/w), gewaschen. Darauf folgten zwei Wäschen in je ml 300 Ethanol. Abschließend wurde das Produkt in Wasser gelöste, der pH-Werte auf 8 eingestellt und über einen Zeitraum von 48 Stunden dialysiert und durch Gefriertrocknung getrocknet.

### Beispiel 4

5 g (atro) einer Hydroxypropylcellulose (MS = 2,8) wurden in 150 ml DMF gegeben und 3 Stunden bei 85°C bis zur vollständigen Lösung gerührt.

Die Sulfatierung wurde durch die Zugabe von 4,1 ml Chlorsulfonsäure (4 mollmol AGU) in 40 ml DMF gestartet. Die Synthese erfolgte bei einer Temperatur von 25°C.

Nach 3 Stunden erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1 Stunden gerührt. Anschließend wurde filtriert und dreimal mit je 300 ml einer Waschlösung, bestehend aus 4% (w/w) Natriumacetat in Ethanol-Wasser-Gemisch (1: 1, w/w), gewaschen. Darauf folgten zwei Wäschen in je 300 ml Ethanol. Abschließend wurde das Produkt in Wasser gelöste, der pH-Werte auf 8 eingestellt und über einen Zeitraum von 48 Stunden dialysiert und durch Gefriertrocknung getrocknet.

### Beispiel 5

5 g (atro) einer Hydroxyethylcellulose (MS = 3,3) wurden in 150 ml DMF gegeben und 3 Stunden bei 85°C bis zur vollständigen Lösung gerührt.

Die Sulfatierung wurde durch die Zugabe von 6,8 g Schwefelsäuretrioxid-TrimethylaminKomplex (3mol/mol AGU) gelöst in 150 ml DMF gestartet. Die Synthese erfolgte bei einer Temperatur von 60°C.

Nach 2 Stunden erfolgte die Fällung unter ständigem Rühren durch langsames gießen der Polymerlösung (innerhalb von 10min.) in ein raumtemperiertes Fällungsmedium, welches sich aus 34 g Natriumhydroxid (NaOH), 64 g H20 und 32 g Natriumacetat aufgefüllt auf 1200 ml Ethanol zusammensetzte. Nach abgeschlossener Fällung wurde noch 2 Stunden gerührt. Anschließend wurde filtriert und dreimal mit je 300 ml einer Waschlösung, bestehend aus 4% (w/w) Natriumacetat in Ethanol-Wasser-Gemisch (1: 1, w/w), gewaschen. Darauf folgten zwei Wäschen in je 300 ml Ethanol. Abschließend wurde das Produkt in Wasser gelöste, der pH-Werte auf 8 eingestellt und über einen Zeitraum von 48 Stunden dialysiert und durch Gefriertrocknung getrocknet.

### Beispiel 6

5 g (atro) einer Hydroxyethylcellulose (MS = 2,5) wurden in 150 ml DMF gegeben und 3 Stunden bei 85°C bis zur vollständigen Lösung gerührt.

Die Sulfatierung wurde durch die Zugabe von 12,5 g Sulfaminsäure (7 mol/mol AGU) gelöst in 100 ml DMF gestartet. Die Synthese erfolgte bei einer Temperatur von 50°C.

Nach 2 Stunden erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1 Stunden gerührt. Anschließend wurde filtriert und dreimal mit je 300 ml einer Waschlösung, bestehend aus 4% (w/w) Natriumacetat in Ethanol-Wasser-Gemisch (1:1, w/w), gewaschen. Darauf folgten zwei Wäschen in je 300 ml Ethanol. Abschließend wurde das Produkt in Wasser gelöste, der pH-Werte auf 8 eingestellt und über einen Zeitraum von 48 Stunden dialysiert und durch Gefriertrocknung getrocknet.

### Beispiel 7

5 g (atro) einer Methylcellulose (DS = 1,94) wurden in 150 ml DMF gegeben und 3 Stunden bei 85°C bis zur vollständigen Lösung gerührt.

Die Sulfatierung wurde durch die Zugabe von 3,5 ml Chlorsulfonsäure (2 mol/mol AGU) in 100 ml DMF gestartet. Die Synthese erfolgte bei einer Temperatur von 25°C.

Nach einer Stunde erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1 Stunden gerührt. Anschließend wurde filtriert und dreimal mit je 300 ml einer Waschlösung, bestehend aus 4% (w/w) Natriumacetat in Ethanol-Wasser-Gemisch (1 :1, w/w), gewaschen. Darauf folgten zwei Wäschen in je 300 ml Ethanol. Abschließend wurde das Produkt in Wasser gelöste, der pH-Werte auf 8 eingestellt und über einen Zeitraum von 48 Stunden dialysiert und durch Gefriertrocknung getrocknet.

### Beispiel 8

5 g (atro) einer Hydroxypropylcellulose (MS = 2,8) wurden in 150 ml DMF gegeben und 3 Stunden bei 85°C bis zur vollständigen Lösung gerührt.

Die Sulfatierung wurde durch die Zugabe von 5,1 ml Chlorsulfonsäure (5 mol/mol AGU) in 40 ml DMF gestartet. Die Synthese erfolgte einer Temperatur von 25°C.

Nach 3 Stunden erfolgte die Fällung unter ständigem Rühren durch langsames gießen der Polymerlösung (innerhalb von 10min.) in ein raumtemperiertes Fällungsmedium, welches sich aus 21 g Natriumhydroxid (NaOH), 40 g H20 und 20 g Natriumacetat aufgefüllt auf 750 ml Ethanol zusammensetzte. Nach abgeschlossener Fällung wurde noch 1 Stunden gerührt. Anschließend wurde filtriert und dreimal mit je 300 ml einer Waschlösung, bestehend aus 4% (w/w) Natriumacetat in Ethanol-Wasser-Gemisch (1:1, w/w), gewaschen. Darauf folgten zwei Wäschen in je 300 ml Ethanol. Abschließend wurde das Produkt in Wasser gelöste, der pH-Werte auf 8 eingestellt und über einen Zeitraum von 48 Stunden dialysiert und durch Gefriertrocknung getrocknet.

In der Tabelle 1 sind tabellarisch die Eigenschaften der Celluloseestersulfate gemäß den Beispielen 1 bis 8 aufgeführt. Die Viskosität und Trübung wurden hierbei in 1-prozentigen (w/w)-Lösung gemessen.

**Tabelle 1**

| **Probe** | **Ausbeute [g]** | **DS** | **Viskosität v [mm^{2/s}]** | **Trübung [NTU]** | **Kommentar Kapselbildung** |
|---|---|---|---|---|---|
| **Beispiel 1** | 2,7 | 0,3 | 116 | 5 | klar, rund |
| **Beispiel 2** | 3,4 | 0,9 | 220 | 3 | klar, leicht verformt |
| **Beispiel 3** | 3,0 | 0,3 | 166 | 4 | klar, rund |
| **Beispiel 4** | 5,4 | 2,3 | 98 | 3 | trüb, rund |
| **Beispiel 5** | 4,2 | 1,8 | 28 | 7 | trüb, rund |
| **Beispiel 6** | 3,8 | 2,95 | 23 | 6 | klar, leicht verformt |
| **Beispiel 7** | 2,6 | 0,7 | 12 | 7 | trüb, rund |
| **Beispiel8** | 5,4 | 2,1 | 53 | 3 | trüb, rund |

## Patentansprüche

1. Verfahren zur Herstellung von sulfatierten Celluloseethern mit folgenden Schritten:
a) Lösen mindestens eines Celluloseethers in einem polaren aprotischen Lösungsmittel,
b) Zusatz eines Sulfatierungsmittels zur Lösung des Celluloseethers, wobei die Sulfatierung bei einer Temperatur von -10 bis 150 °C erfolgt,
c) Ausfällung des sulfatierten Zwischenprodukts in einem Fällungsmedium enthaltend Wasser, mindestens einen Alkohol und mindestens ein Natriumsalz,
d) Waschen und Trocknen des Fällungsprodukts unter Erhalt eines Celluloseethersulfats.

2. Verfahren nach Anspruch 1, **dadurch gekennzeichnet, dass** der mindestens eine Celluloseether ausgewählt ist aus der Gruppe bestehend aus Methylcellulose, Hydroxyethylcellulose, Hydroxypropylcellulose, Ethylcellulose, Methylhydroxyethylcellulose, Methylhydroxypropylcellulose, Methylhydroxybutylcellulose, Ethylhydroxyethylcellulose, Carboxymethylhydroxyethylcellulose, Benzylcellulose oder Mischungen hiervon.

3. Verfahren nach einem der vorhergehenden Ansprüche, **dadurch gekennzeichnet, dass** das polare aprotische Lösungsmittel ausgewählt ist aus der Gruppe bestehend aus
- tertiäre Carbonsäureamide, insbesondere Dimethylformamid,
- Kohlesäureester, insbesondere Dimethylcarbonat,
- Sulfoxide, insbesondere Dimethylsulfoxid,
- Lactame, insbesondere N-Methyl-2-pyrrolidon sowie
- Mischungen hiervon.

4. Verfahren nach einem der vorhergehenden Ansprüche, **dadurch gekennzeichnet, dass** das Sulfatierungsmittel ausgewählt ist aus der Gruppe bestehend aus Schwefelsäure, Chlorsulfonsäure, Amidosulfonsäure, SO₃-Komptexe und Mischungen hiervon.

5. Verfahren nach einem der vorhergehenden Ansprüche, **dadurch gekennzeichnet, dass** das Fällungsmedium als Alkohol Methanol, Ethanol, Propanol, 2-Propanol oder 2-Methyl-2-Propanol und Mischungen hiervon enthält.

6. Verfahren nach einem der vorhergehenden Ansprüche, **dadurch gekennzeichnet, dass** das Fällungsmedium als Natriumsalz Natriumacetat, Natriumchlorid, Natriumhydrogencarbonat, Natriumcarbonat, Natriumsulfat enthält.

7. Verfahren nach einem der vorhergehenden Ansprüche,
**dadurch gekennzeichnet, dass** das Waschen mit einem Alkohol, insbesondere Methanol, Ethanol, Propanol, 2-Propanol oder 2-Methyl-2-Propanol und/oder das Trocknen mittels Gefriertrocknung, Trocknung im Vakuum, Sprühtrocknung und/oder Lösungsmittelaustausch erfolgt.

8. Sulfatierter Celluloseester herstellbar nach dem Verfahren nach einem der vorhergehenden Ansprüche mit einem DS_{(Sulfat)} von 0,1 bis 2,9 und einer Lösungsviskosität im Bereich von 1 bis 1000 mPas bei einer Konzentration des Celluloseethersulfats von 1 % (w/w) in Wasser.

9. Verfahren zur Herstellung von Mikrokapseln aus mindestens einem sulfatierten Celluloseester nach Anspruch 8 und mindestens einem kationischen Polymer mit folgenden Schritten:
i) Herstellung einer wässrigen Lösung des sulfatierten Celluloseesters, wobei die Konzentration des sulfatierten Celluloseesters im Bereich von 0,5 bis 10 % (w/w) liegt,
ii) Zugabe eines mindestens eines zu verkapselnden Materials,
iii) Vertropfung der Lösung aus ii) in eine kationische Polymerlösung unter Bildung von Mikrokapseln.

10. Verfahren nach Anspruch 9, **dadurch gekennzeichnet, dass** die kationische Polymerlösung Dodecylamin, Ethylenamin, Piperazin, Methylblau, Arginin, Triethylamin, Spermin, Polydimethylammonium, Polyvinylbenzyltrimethylammonium, kationische Chitosane, deren Derivate sowie Mischungen hiervon enthält.

11. Verfahren nach einem der Ansprüche 9 oder 10, **dadurch gekennzeichnet, dass** mindestens ein zu verkapselndes Material biologischen Ursprungs ist und bevorzugt ausgewählt ist aus der Gruppe bestehend aus nativen oder veränderten Zellen von Mensch und Tier, native oder veränderte Bakterien native oder veränderte Viren, native oder veränderte Hefen, isolierte Proteine oder Proteingemische, Enzymen, Antikörper oder Antikörperbestandteile und/oder Nukleinsäuremoleküle.

12. Verfahren nach einem der Ansprüche 9 bis 11, **dadurch gekennzeichnet, dass** mindestens ein zu verkapselndes Material nicht-biologischen Ursprungs ist und bevorzugt ausgewählt ist aus der Gruppe bestehend aus Farbstoffen, Geschmackstoffen, Geschmacksverstärkern, Aromen, Duftstoffen, Insektiziden, Pestiziden, Fungiziden, Detergenzien und Kombinationen hiervon.

13. Mikrokapseln herstellbar nach dem Verfahren nach einem der Ansprüche 9 bis 12.

14. Verwendung der Mikrokapseln nach Anspruch 13 als Arzneimittel, insbesondere zur Implantation oder Injektion.

## Claims

1. A method for the preparation of sulfated cellulose ethers, with the following steps:
a) dissolving at least one cellulose ether in a polar aprotic solvent,
b) adding a sulfating agent to the solution of the cellulose ether, wherein the sulfating takes place at a temperature of -10 to 150°C,
c) precipitating the sulfated intermediate product in a precipitation medium containing water, at least one alcohol and at least one sodium salt,
d) washing and drying the precipitation product, with a cellulose ether sulfate being obtained.

2. A method according to Claim 1, **characterised in that** the at least one cellulose ether is selected from the group consisting of methylcellulose, hydroxyethylcellulose, hydroxypropylcellulose, ethylcellulose, methylhydroxyethylcellulose, methylhydroxypropylcellulose, methylhydroxybutylcellulose, ethylhydroxyethylcellulose, carboxymethylhydroxyethylcellulose, benzylcellulose or mixtures thereof.

3. A method according to one of the preceding claims, **characterised in that** the polar aprotic solvent is selected from the group consisting of
- tertiary carboxylic acid amides, in particular dimethyl formamide,
- carbonic acid esters, in particular dimethyl carbonate,
- sulfoxides, in particular dimethyl sulfoxide,
- lactams, in particular N-methyl-2-pyrrolidone, and also
- mixtures thereof.

4. A method according to one of the preceding claims, **characterised in that** the sulfating agent is selected from the group consisting of sulfuric acid, chlorosulfonic acid, amidosulfonic acid, SO₃ complexes and mixtures thereof.

5. A method according to one of the preceding claims, **characterised in that** the precipitation medium contains as alcohol methanol, ethanol, propanol, 2-propanol or 2-methyl-2-propanol and mixtures thereof.

6. A method according to one of the preceding claims, **characterised in that** the precipitation medium contains as sodium salt sodium acetate, sodium chloride, sodium hydrogen carbonate, sodium carbonate or sodium sulfate.

7. A method according to one of the preceding claims, **characterised in that** the washing takes place with an alcohol, in particular methanol, ethanol, propanol, 2-propanol or 2-methyl-2-propanol, and/or the drying takes place by means of freeze-drying, drying in a vacuum, spray-drying and/or solvent exchange.

8. A sulfated cellulose ester which can be produced according to the method according to one of the preceding claims with a DS_{(sulfate)} of 0.1 to 2.9 and a solution viscosity in the range from 1 to 1000 mPas at a concentration of the cellulose ether sulfate of 1% (w/w) in water.

9. A method for producing microcapsules from at least one sulfated cellulose ester according to Claim 8 and at least one cationic polymer, having the following steps:
i) producing an aqueous solution of the sulfated cellulose ester, wherein the concentration of the sulfated cellulose ester lies in the range from 0.5 to 10% (w/w),
ii) adding an at least one material to be encapsulated,
iii) dropping the solution from ii) into a cationic polymer solution, forming microcapsules.

10. A method according to Claim 9, **characterised in that** the cationic polymer solution contains dodecylamine, ethyleneamine, piperazine, methyl blue, arginine, triethylamine, spermine, polydimethylammonium, polyvinylbenzyltrimethylammonium, cationic chitosans, derivatives thereof and mixtures thereof.

11. A method according to one of Claims 9 or 10, **characterised in that** the at least one material to be encapsulated is of biological origin and preferably is selected from the group consisting of naturally-occurring or modified cells of humans and animals, naturally-occurring or modified bacteria, naturally-occurring or modified viruses, naturally-occurring or modified yeasts, isolated proteins or protein mixtures, enzymes, antibodies or antibody constituents and/or nucleic acid molecules.

12. A method according to one of Claims 9 to 11, **characterised in that** the at least one material to be encapsulated is of non-biological origin and preferably is selected from the group consisting of dyes, flavourings, flavour enhancers, flavours, fragrances, insecticides, pesticides, fungicides, detergents and combinations thereof.

13. Microcapsules which can be produced according to the method according to one of Claims 9 to 12.

14. Use of the microcapsules according to Claim 13 as medicaments, in particular for implantation or injection.

## Revendications

1. Procédé de préparation d'éthers de cellulose sulfatés, comportant les étapes suivantes :
a) dissolution d'au moins un éther de cellulose dans un solvant aprotique polaire,
b) ajout d'un agent de sulfatation à la solution de l'éther de cellulose, la sulfatation étant mise en œuvre à une température de -10 à 150 °C,
c) précipitation du produit intermédiaire sulfaté dans un milieu de précipitation contenant de l'eau, au moins un alcool et au moins un sel de sodium,
d) lavage et séchage du produit de la précipitation, avec obtention d'un éthersulfate de cellulose.

2. Procédé selon la revendication 1, **caractérisé en ce que** l'au moins un éther de cellulose est choisi dans le groupe consistant en la méthylcellulose, l'hydroxyéthylcellulose, l'hydroxypropylcellulose, l'éthylcellulose, la méthylhydroxyéthylcellulose, la méthylhydroxypropylcellulose, la méthylhydroxybutylcellulose, l'éthylhydroxyéthylcellulose, la carboxyméthylhydroxyéthylcellulose, la benzylcellulose ou les mélanges de celles-ci.

3. Procédé selon l'une des revendications précédentes, **caractérisé en ce que** le solvant aprotique polaire est choisi dans le groupe consistant en
- les carboxamides tertiaires, en particulier le diméthylformamide,
- les esters de l'acide carbonique, en particulier le carbonate de diméthyle,
- les sulfoxydes, en particulier le diméthylsulfoxyde,
- les lactames, en particulier la N-méthyl-2-pyrrolidone, ainsi que
- les mélanges de ceux-ci.

4. Procédé selon l'une des revendications précédentes, **caractérisé en ce que** l'agent de sulfatation est choisi dans le groupe consistant en l'acide sulfurique, l'acide chlorosulfonique, l'acide amidosulfonique, les complexes de SO₃ et les mélanges de ceux-ci.

5. Procédé selon l'une des revendications précédentes, **caractérisé en ce que** le milieu de précipitation contient en tant qu'alcool du méthanol, de l'éthanol, du propanol, du 2-propanol ou du 2-méthyl-2-propanol, et des mélanges de ceux-ci.

6. Procédé selon l'une des revendications précédentes, **caractérisé en ce que** le milieu de précipitation contient en tant que sel de sodium de l'acétate de sodium, du chlorure de sodium, de l'hydrogénocarbonate de sodium, du carbonate de sodium, du sulfate de sodium.

7. Procédé selon l'une des revendications précédentes, **caractérisé en ce que** le lavage est mis en œuvre avec un alcool, en particulier le méthanol, l'éthanol, le propanol, le 2-propanol ou le 2-méthyl-2-propanol, et/ou le séchage est mis en œuvre par lyophilisation, séchage sous vide, séchage par atomisation et/ou échange de solvants.

8. Ester de cellulose sulfaté pouvant être préparé par le procédé selon l'une des revendications précédentes, présentant un DS_{(sulfate)} de 0,1 à 2,9 et une viscosité en solution comprise dans la plage de 1 à 1000 mPa.s pour une concentration de l'éthersulfate de cellulose de 1 % (p/p) dans l'eau.

9. Procédé de fabrication de microcapsules à partir d'au moins un ester de cellulose sulfaté selon la revendication 8 et d'au moins un polymère cationique, comportant les étapes suivantes :
i) préparation d'une solution aqueuse de l'ester de cellulose sulfaté, la concentration de l'ester de cellulose sulfaté étant comprise dans la plage de 0,5 à 10 % (p/p),
ii) ajout d'au moins un matériau à encapsuler,
iii) transformation en gouttes de la solution de ii) dans une solution d'un polymère cationique avec formation de microcapsules.

10. Procédé selon la revendication 9, **caractérisé en ce que** la solution d'un polymère cationique contient de la dodécylamine, de l'éthylène-amine, de la pipérazine, du bleu de méthyle, de l'arginine, de la triéthylamine, de la spermine, du polydiméthylammonium, du polyvinylbenzyltriméthylammonium, des chitosanes cationiques, leurs dérivés, ainsi que les mélanges de ceux-ci.

11. Procédé selon l'une des revendications 9 ou 10, **caractérisé en ce qu'**au moins un matériau à encapsuler est d'origine biologique et est de préférence choisi dans le groupe consistant en les cellules natives ou modifiées d'origine humaine et animale, les bactéries natives ou modifiées, les virus natifs ou modifiés, les levures natives ou modifiées, les protéines ou mélanges de protéines isolées, les enzymes, les anticorps ou les constituants d'anticorps et/ou les molécules d'acides nucléiques.

12. Procédé selon l'une des revendications 9 à 11, **caractérisé en ce qu'**au moins un matériau à encapsuler est d'origine non biologique et est de préférence choisi dans le groupe consistant en les colorants, les agents de sapidité, les exhausteurs de goût, les aromatisants, les parfums, les insecticides, les pesticides, les fongicides, les détergents et les combinaisons de ceux-ci.

13. Microcapsules pouvant être fabriquées par le procédé selon l'une des revendications 9 à 12.

14. Utilisation des microcapsules selon la revendication 13 en tant que médicament, en particulier pour implantation ou injection.
